(19) Europäisches Patentamt
European Patent Office
Office européen des brevets

(11) Veröffentlichungsnummer: **0 026 321**
**B1**

(12) # EUROPÄISCHE PATENTSCHRIFT

(45) Veröffentlichungstag der Patentschrift: **09.02.83**

(51) Int. Cl.³: **C 07 D 403/04**, A 61 K 31/50

(21) Anmeldenummer: **80104977.6**

(22) Anmeldetag: **21.08.80**

(54) **6-Imidazol-1-yl-3-hydrazino-pyridazine, Verfahren zu ihrer Herstellung und diese enthaltende Arzneimittel.**

(30) Priorität: **01.09.79 DE 2935359**

(43) Veröffentlichungstag der Anmeldung: **08.04.81 Patentblatt 81/14**

(45) Bekanntmachung des Hinweises auf die Patenterteilung: **09.02.83 Patentblatt 83/6**

(84) Benannte Vertragsstaaten: **AT BE CH DE FR GB IT LI NL SE**

(56) Entgegenhaltungen:
**DE-A-2 825 906**
**DE-A-2 831 072**
**US-A-3 642 792**
**EUROPEAN JOURNAL OF MEDICINAL CHEMISTRY, Band 14, Nr. 5, September/Oktober 1979 G. SZILAGYI et al. »Studies in the Field of Pyridazine Compounds, III (1). Hypotensive 3-(1-pyrazolyl) Pyridazine Derivatives« Seiten 439 bis 445**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**
Erfinder: **Gries, Josef, Dr., Roemerweg 43, D-6706 Wachenheim (DE)**
Erfinder: **Lenke, Dieter, Dr., Kekuleplatz 1, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 026 321 B1

BUNDESDRUCKEREI BERLIN

6-Imidazol-1-yl-3-hydrazino-pyridazine, Verfahren zu ihrer Herstellung und diese enthaltende Arzneimittel

Die Erfindung betrifft gegebenenfalls im Imidazolring durch Halogenatome oder bestimmte Alkylgruppen substituierte 6-Imidazol-1-yl-3-hydrazino-pyridazine und ihre physiologisch verträglichen Säureadditionssalze, Verfahren zu ihrer Herstellung und diese enthaltende Arzneimittel zur Behandlung der Hypertonie.

In der US-A-3 642 792 sind bereits verschiedene Hydrazinopyridazine bekannt, welche mit gewissen 6gliedrigen heterocyclischen gesättigten Ringen substituiert sind und hypotensive Aktivitäten aufweisen.

3-Hydrazino-pyridazine, die in 6-Stellung durch einen Pyrazolylring substituiert sind, sind als Verbindungen mit blutdrucksenkenden Eigenschaften beschrieben worden (Eur. J. Med. Chem. 14, 439–445 (1979)). Ebenso sind aus der DE-A-2 831 072 gewisse Pyrazolyl-hydrazino-pyridazine mit hypotensiver Wirkung bekannt. Demgegenüber wurde nun gefunden, daß 6-Imidazolyl-3-hydrazino-pyridazine der allgemeinen Formel I

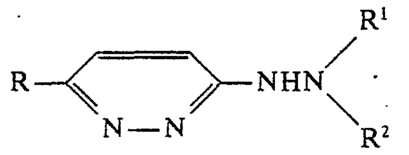

(I)

in der R einen Imidazol-1-yl-rest, der gegebenenfalls ein- oder zweifach durch Halogenatome oder ein- bis dreifach durch Alkylgruppen mit 1 bis 3 C-Atomen substituiert ist, bedeutet und für $R^1$ und $R^2$ je ein Wasserstoffatom steht, oder $R^1$ und $R^2$ bilden zusammen den Rest

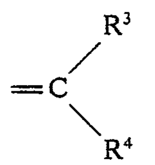

in dem $R^3$ und $R^4$ einen Alkylrest mit 1 bis 4 Kohlenstoffatomen oder einen Alkenylrest mit 2 bis 5 Kohlenstoffatomen bedeuten, und ihre physiologisch verträglichen Säureadditionssalze besonders wertvolle pharmakologische Eigenschaften, insbesondere eine ausgeprägte blutdrucksenkende Wirkung, aufweisen.

Halogenatome im Imidazolrest sind insbesondere Chloratome. Bevorzugte Verbindungen sind in 2-Stellung alkylierte Imidazol-1-yl-reste. Hiervon seien im einzelnen beispielsweise genannt 2-Methyl-imidazol-1-yl, 2-Äthyl-imidazol-1-yl, 4-Methyl-imidazol-1-yl, 2,4,5-Trimethyl-imidazol-1-yl und 2-Isopropyl-imidazol-1-yl.

Im Hinblick auf den Rest

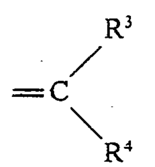

sind Verbindungen der Formel I, in denen $R^3$ und $R^4$ Methylgruppen und Verbindungen, in denen $R^3$ gleich Methyl und $R^4$ gleich 2-Methyl-1-propenyl ist, hervorzuheben.

Die Verbindungen der Formel I werden hergestellt, indem man das Alkali- oder Erdalkalimetall-Salz eines am N-Atom 1 unsubstituierten Imidazols entsprechend dem Rest R der Formel I in einem hochsiedenden polaren aprotischen Lösungsmittel bei Temperaturen von 20 bis 170°C mit 3,6-Dichlorpyridazin umsetzt und das erhaltene 3-Chlor-6-(imidazol-1-yl)-pyridazin anschließend mit Hydrazinhydrat gegebenenfalls in Gegenwart eines Lösungsmittels bei Temperaturen von 80°C bis zu Rückflußtemperaturen des Reaktionsgemisches zu einem 6-(Imidazol-1-yl)-3-hydrazino-pyridazin der allgemeinen Formel I umsetzt und gegebenenfalls die erhaltene Hydrazinoverbindung mit einer Carbonylverbindung der Formel

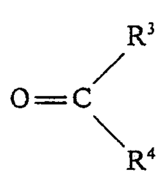

in der $R^3$ und $R^4$ die oben genannten Bedeutungen haben, zu einem Hydrazon umsetzt und gegebenenfalls die erhaltene Verbindung der Formel I in ein Säureadditionssalz einer physiologisch verträglichen Säure überführt.

Die Umsetzung wird durch das folgende Reaktionsschema veranschaulicht:

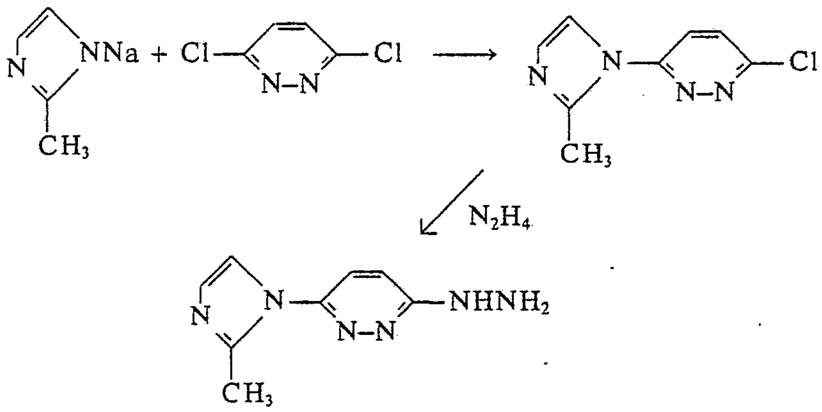


(I)

Das Imidazol wird dabei vorteilhafterweise durch Zugabe einer äquivalenten Menge einer starken Base, die geeignet ist, unter Salzbildung zu reagieren, in ein Alkali- oder Erdalkalisalz überführt. Geeignete Basen sind z. B. alkali- bzw. Erdalkali-Hydride, -Amide oder metallorganische Verbindungen, vorzugsweise Natriumhydrid. Die Salzbildung wird zweckmäßig in einem hochsiedenden polaren aprotischen Lösungsmittel, vorzugsweise Dimethylformamid, bei Temperaturen von 20 bis 80°C und halb- bis zweistündigem Nachrühren vorteilhaft unter Schutzgasatmosphäre durchgeführt. Anschließend läßt man mit 1 Mol-Äquivalent 3,6-Dichlorpyridazin bei Temperaturen von 20 bis 170°C in der Regel innerhalb von 3 bis 10 Stunden zu dem entsprechenden 3-Chlor-6-(imidazol-1-yl)-pyridazin reagieren.

Die Reinigung des 3-Chlor-6-imidazolyl-pyridazins erfolgt vorteilhaft durch Säulenchromatographie an Kieselgel, mit einem Gemisch aus Methylenchlorid/Methanol (70 – 90 : 30 – 10 Volumenteile) als Laufmittel in an sich üblicher Weise.

Das erhaltene 3-Chlor-6-(imidazol-1-yl)-pyridazin wird dann mit überschüssigem Hydrazinhydrat, gegebenenfalls in einem polaren protischen Lösungsmittel, wie Äthanol, Isopropanol oder Methanol, innerhalb von 3 bis 6 Stunden bei Temperaturen von 80°C bis zu den Siedetemperaturen zu den erfindungsgemäßen 6-(Imidazol-1-yl)-3-hydrazinopyridazinen der Formel I umgesetzt.

Das Endprodukt kann durch Umkristallisation aus einem niederen Alkohol oder durch Säulenchromatographie gereinigt werden.

Die erfindungsgemäßen Verbindungen der allgemeinen Formel I, in denen $R^1$ und $R^2$ den Rest

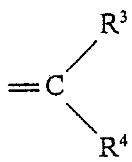

bedeuten, erhält man durch Überführen der freien Hydrazine in die entsprechenden Hydrazone in an sich üblicher Weise, wie das folgende Formelbild zeigt:

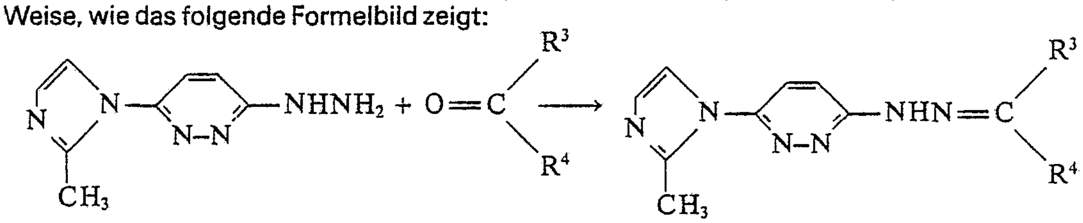

Ein 6-(Imidazol-1-yl)-3-hydrazino-pyridazin wird mit einer geeigneten Carbonylverbindung, wie Aceton, Acetaldehyd, Benzaldehyd, Furfural, Mesityloxid, Diallylketon etc., in bekannter Weise zum entsprechenden Hydrazon kondensiert, gegebenenfalls in einen niederen Alkohol, insbesondere Äthanol, als Lösungsmittel und in Gegenwart einer katalytischen Menge Säure, wie Essigsäure oder Salzsäure.

Die erfindungsgemäßen Verbindungen werden im allgemienen als farblose bis gelbbraune Kristalle erhalten und können aus den üblichen organischen Lösungsmitteln, bevorzugt aus Isopropanol oder Äthanol, umkristallisiert oder säulenchromatographisch, wie oben bei den Zwischenprodukten

angegeben, gereinigt werden. Die erfindungsgemäßen Verbindungen bilden mit pharmakologisch verträglichen anorganischen und organischen Säuren nichttoxische Säureadditionssalze. Die Säureadditionssalze werden beispielsweise durch Versetzten der freien Base mit ein oder zwei Äquivalenten einer Säure, vorzugsweise in einem neutralen Lösungsmittel, in an sich üblicher Weise erhalten.

Geeignete physiologisch verträgliche Säuren können beispielsweise Fortschritte der Arzneimittelforschung Band 10, Seiten 224 bis 225, Birkhäuser Verlag, Basel und Stuttgart, 1966 oder dem Journal of Pharmaceutical Science, Vol. 66, Seiten 1 bis 5 (1977) entnommen werden.

Neben den in den Beispielen aufgeführten Verbindungen seien beispielsweise noch genannt:

3-(2-Isopropyliden-hydrazino)-6-(2,4,5-trimethyl-imidazol-1-yl)-pyridazin,
3-(2-[2-Methyl-2-penten-4-yliden]hydrazino)-6-(2-methyl-imidazol-1-yl)-pyridazin,
3-(2-[1,6-Heptadien-4-yliden]hydrazino)-6-(2-methyl-imidazol-1-yl)-pyridazin,
3-(2-[2-Methyl-2-penten-4-yliden]hydrazino)-6-(2-äthyl-imidazol-1-yl)-pyridazin,
3-Hydrazino-6-(2-isopropyl-imidazol-1-yl)-pyridazin,
3-(2-Isopropyliden-hydrazino)-6-(2-isopropyl-imidazol-1-yl)-pyridazin.

Die erfindungsgemäßen Verbindungen der allgemeinen Formel I sowie deren pharmakologisch verträgliche Säureadditionssalze besitzen ausgeprägte blutdrucksenkende Eigenschaften und können als Mittel bei der Behandlung der Hypertonie verwendet werden.

Als besonders wirksam hervorzuheben sind:

3-Hydrazino-6-(2-methyl-imidazol-1-yl)-pyridazin,
3-(2-Isopropyliden-hydrazino)-6-(2-methyl-imidazol-1-yl)-pyridazin,
3-Hydrazino-6-(2-äthyl-imidazol-1-yl)-pyridazin,
3-(2-Isopropyliden-hydrazino)-6-(2-äthyl-imidazol-1-yl)-pyridazin.

Die blutdrucksenkende Wirkung wurde an narkotisierten Ratten (Stamm: Sprague Dawley, Gewicht: 230 bis 280 g) in Urethan-Narkose (1,78 g/kg i. p.) nachgewiesen. Der Blutdruck wurde in der Aorta carotis gemessen. Die Substanzapplikation erfolgte in die Vena jugularis (wäßrige Lösung 1 ml/kg). Als ED 20% wurden aus der linearen Regression zwischen log Dosis (mg/kg) und relativer Blutdrucksenkung ($\Delta$%) die Dosen ermittelt, welche eine 20%ige Blutdrucksenkung bewirken.

Die antihypertensive Wirkung wurde an spontan hypertonen Ratten (Stamm: Okamoto) ermittelt. Die Substanzen wurden Gruppen von 4 bis 8 männlichen Tieren oral appliziert. Vor und 2 h nach der Applikation wurde der systolische Blutdruck am Rattenschwanz mit Hilfe von Piezokristallaufnehmern gemessen. Als ED 20% wurden unter Berücksichtigung der Werte Placebo-behandelter Kontrolltiere aus den linearen Regressionen zwischen log Dosis (mg/kg) und relativer Blutdrucksenkung ($\Delta$%) die Dosen bestimmt, welche den systolischen Druck um 20% senken.

Zur Bestimmung der akuten Toxität (LD 50) wurden die Substanzen Gruppen von je 10 weiblichen NMRI-Mäusen (Gewicht 20 bis 25 g) intraperitoneal appliziert. Als ED 50 wurde die Dosis berechnet (Probit-Analyse), nach der 50% der Tiere innerhalb von 7 Tagen starben.

Tabelle 1

Hypotensive und antihypertensive Wirkung sowie Toxizität

| Verbindung Beispiel Nr. | Hypotensive Wirkung[1]) ED 20% | | Antihypertensive Wirkung[2]) ED 20% | | Toxizität[3]) LD 50 | | Therap. Breite Q |
|---|---|---|---|---|---|---|---|
| | mg/kg | R. W.[4]) | mg/kg | R. W. | mg/kg | R. W. | |
| 1 a | 0,0310 | 2,75 | 1,41 | 4,86 | 193 | 1,06 | 137 |
| 1 c | 0,0638 | 1,34 | 2,91 | 2,35 | 156 | 1,32 | 53,6 |
| 2 a | 0,0464 | 1,84 | 2,46 | 2,78 | 98,6 | 2,09 | 40,1 |
| 2 b | 0,0464 | 1,84 | 2,48 | 2,76 | 124 | 1,66 | 50,1 |
| Dihydralazin | 0,0854 | 1,00 | 6,85 | 1,00 | 206 | 1,00 | 30,1 |

[1]) Narkotisierte Ratte, Applikation: intravenös.
[2]) Spontan hypertone Ratte (SHR), Applikation: per os.
[3]) Maus, Applikation: intraperitoneal.
[4]) R. W. = Relative Wirksamkeit; Dihydralazin = 1,00.

[5]) $Q = \frac{\text{LD 50 mg/kg intraperitoneal}}{\text{ED 20\% mg/kg per os}}$

Wie aus der Tabelle zu entnehmen ist, senken die erfindungsgemäßen Verbindungen den Blutdruck narkostisierter Ratten in Dosen, die 1,3 (Beispiel 1c) bis 2,8mal (Beispiel 1a) kleiner sind als die von Dihydralazin. An wachen spontan hypertonen Ratten sind die Substanzen 2,4 mal (Beispiel 1c) bis 4,9mal (Beispiel 1a) wirksamer als Dihydralazin. Die toxischen Dosen (LD 50) sind 40- (Beispiel 2a) bis 137mal (Beispiel 1a) größer als die antihypertensiven Dosen (bei Dihydralazin 30mal).

Gegenstand der vorliegenden Erfindung ist demnach auch ein therapeutisches Mittel, gekennzeichnet durch einen Gehalt an einer Verbindung der Formel I oder deren pharmakologisch verträgliches Säureadditionssalz als Wirkstoff neben üblichen Trägerstoffen oder Verdünnungsmitteln, sowie die Verbindungen der Formel 1 zur Verwendung zu therapeutischen Zwecken.

Die therapeutischen Mittel oder Zubereitungen werden mit den pharmazeutisch üblichen Trägerstoffen oder Verdünnungsmitteln und den gegebenenfalls üblicherweise verwendeten pharmazeutisch-technischen Hilfsstoffen entsprechend der gewünschten Applikationsart und mit einer zur Anwendung geeigneten Dosiereinheit in an sich üblicher Weise, insbesondere durch Vermischen, hergestellt. Dabei kommen beim Menschen Dosen von 20 bis 200 mg, bevorzugt 50 bis 150 mg, in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäßen Verbindungen mit den in der galenischen Pharmazie üblichen flüssigen oder festen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen (vgl. L. G. Goodman, A. Gilman, The Pharmaceutical Basis of Therapeutics).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die folgenden Beispiele dienen zur Erläuterung der Erfindung:

## Herstellung von Ausgangsverbindungen

### Beispiel I

3-Chlor-6-(2-methyl-imidazol-1-yl)-pyridazin

8,55 g (104 mM) 2-Methylimidazol in 80 ml Dimethylformamid werden unter Rühren portionsweise mit 104 mM einer 55%igen Suspension von Natriumhydrid in Paraffinöl versetzt und durch einstündiges Nachrühren bei Raumtemperatur unter Stickstoff als Schutzgas in das Natriumsalz übergeführt. Anschließend gibt man zu dem Reaktionsgemisch portionsweise 15,5 g (104 mM) 3,6-Dichlorpyridazin zu (exotherme Reaktion, Temperaturanstieg auf 50 bis 60°C) und läßt 5 bis 10 Stunden bei Raumtemperatur unter Stickstoff als Schutzgas nachrühren. Nach dem Abkühlen destilliert man das Lösungsmittel im Ölpumpenvakuum bei ca. 0,5 Torr ab, nimmt den Rückstand in Eiswasser auf, extrahiert die wäßrige Lösung mit Methylenchlorid und wäscht die organische Phase mehrmals mit Wasser aus. Die gleiche Ausbeute wird erhalten, wenn man nach der Zugabe des 3,6-Dichlorpyridazins 3 bis 5 Stunden lang auf Rückflußtemperaturen unter Stickstoff als Schutzgas erhitzt. Das erhaltene Rohprodukt wird durch Säulenchromatographie (Kieselgel, Laufmittel Methylenchlorid-Methanol 90/10) gereinigt. Ausbeute: 8,5 g (42%), Kristalle mit Schmp. 104 bis 107° C.

Die Erfahrung zeigt, daß sich noch bessere Ausbeuten bei umgekehrter Zugabe erzielen lassen: man legt das 3,6-Dichlorpyridazin in Dimethylformamid vor und gibt portionsweise das Na-Salz des 2-Methylimidazols in Dimethylformamid unter gutem Rühren hinzu.

Gemäß Beispiel I werden die folgenden Verbindungen hergestellt:

3-Chlor-6-(imidazol-1-yl)-pyridazin, Schmp. 178 bis 180° C,
3-Chlor-6-(2-äthyl-imidazol-1-yl)-pyridazin; Öl,
3-Chlor-6-(2,4,5-trimethyl-imidazol-1-yl)-pyridazin; Öl,
3-Chlor-6-(4-methyl-imidazol-1-yl)-pyridazin,
Schmp. 165 bis 168° C.

## Herstellung von erfindungsgemäßen Verbindungen

### Beispiel 1

a) 3-Hydrazino-6-(2-methyl-imidazol-1-yl)-pyridazin

6,7 g (35 mM) 3-Chlor-6-(2-methyl-imidazol-1-yl)-pyridazin werden in 30 ml Hydrazinhydrat aufgenommen und unter Stickstoff als Schutzgas 5 bis 10 Stunden bei 100 bis 130° C erhitzt, wobei sich das Reaktionsgemisch im Verlauf der ersten Stunden homogenisiert. Nach dem Abkühlen wird das ausgefallene Rohprodukt abgesaugt. Man kann auch in Eiswasser gießen und extrahiert mehrmals mit Methylenchlorid. Als zweckmäßig hat sich die kontinuierliche Extraktion mit Hilfe eines Rotationsperforators und den Extraktions-Lösungsmitteln Methylenchlorid oder Chloroform erwiesen. Man kann auch das Hydrazinhydrat im Vakuum abdestillieren.

Das erhaltene Rohprodukt wird in Isopropanol unter Erwärmen aufgenommen, man engt das Lösungsmittel zweckmäßig ein und läßt in der Kälte auskristallisieren. Das 3-Hydrazino-6-(2-methyl-imidazol-1-yl)-pyridazin wird durch Säulenchromatographie (Kieselgel, Laufmittel Methylenchlorid/Methanol 90/10) gereinigt und mit ehterischer Salzsäure in ethanolischer Lösung ins Hydrochlorid übergeführt.

Ausbeute: Dihydrochlorid 7,0 g (76%), Schmp.: 280 bis 282° C (Zers.). Die freie Base schmilzt bei 154 bis 156° C.

Entsprechend werden die folgenden Verbindungen hergestellt:

b) 3-Hydrazino-6-(imidazol-1-yl)-pyridazin, Schmp. 199 – 202° C,
c) 3-Hydrazino-6-(2-äthyl-imidazol-1-yl)-pyridazin · 0,5 $H_2O$, Schmp. 94 – 97° C,
d) 3-Hydrazino-6-(2,4,6-trimethyl-imidazol-1-yl)-pyridazin · 4 HCl · 2 $H_2O$, Schmp. 64 – 68° C.
e) 3-Hydrazino-6-(4-methyl-imidazol-1-yl)-pyridazin · 2 HCl, Schmp. 257 bis 260° C.

### Beispiel 2

a) 3-(2-Isopropyliden-hydrazino)-6-(2-methyl-imidazol-1-yl)-pyridazin

3,0 g (15,8 mM) 3-Hydrazino-6-(2-methyl-imidazol-1-yl)-pyridazin werden in 40 ml Aceton suspendiert und am Dampfbad 10 Minuten erwärmt. Die heiße Lösung wird anschließend filtriert. Beim Abkühlen kristallisiert das 3-(2-Isopropyliden-hydrazino)-6-(2-methyl-imidazol-1-yl)-pyridazin mit dem Schmp. 173 bis 175° C aus.

Gemäß dieser Vorschrift werden die folgenden Verbindungen hergestellt:

b) 3-3-(2-Isopropyliden-hydrazino)-6-(2-äthyl-imidazol-1-yl)-pyridazin, Schmp. 155 bis 156° C.
c) 3-(2-Isopropyliden-hydrazino)-6-(4-methyl-imidazol-1-yl)-pyridazin, Schmp. 224 bis 226° C.

Beispiele für die pharmazeutische Anwendung

1. Tabletten:

| | |
|---|---|
| Wirkstoff | 60 mg |
| Polyvinylpyrrolidon (mittl. M. G. 25 000) | 170 mg |
| Polyäthylglykol (mittl. M. G. 4 000) | 14 mg |
| Hydroxypropylmethylcellulose | 40 mg |
| Talkum | 4 mg |
| Magnesiumstearat | 2 mg |
| | 290 mg |

Der Wirkstoff wird mit Polyvinylpyrrolidon in 10%iger wäßriger Lösung befeuchtet, durch ein Sieb mit der lichten Maschenweite 1,0 mm getrieben und bei 50° C getrocknet. Dieses Granulat wird mit Polyäthylenglykol (mittl. M. G. 4 000), Hydroxypropylmethylcellulose, Talkum und Magnesiumstearat vermischt und zu Tabletten à 290 mg verpreßt.

2. Beispiel für Dragees:

| | |
|---|---|
| Wirkstoff | 90 mg |
| Lactose | 90 mg |
| Maisstärke | 60 mg |
| Polyvinylpyrrolidon | 6 mg |
| Magnesiumstearat | 1 mg |
| | 247 mg |

Die Mischung der Wirkstoffsubstanz mit Lactose und Maisstärke wird mit einer 8%igen wäßrigen Lösung des Polyvinylpyrrolidons durch ein Sieb 1,5 mm granuliert, bei 50° C getrocknet und nochmals durch ein Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. 6-Imidazolyl-3-hydrazino-pyridazine der Formel I

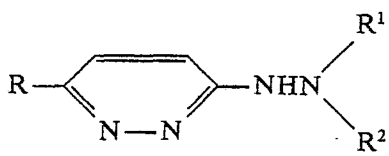

(I)

in der R einen Imidazolrest, der gegebenenfalls ein- oder zweifach durch Halogenatome oder ein- bis dreifach durch Alkylgruppen mit 1 bis 3 C-Atomen substituiert ist, bedeutet und für $R^1$ und $R^2$ je ein Wasserstoffatom steht, oder $R^1$ und $R^2$ bilden zusammen den Rest

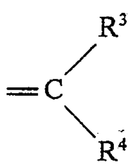

in dem $R^3$ und $R^4$ einen niedrigen Alkylrest mit 1 bis 4 Kohlenstoffatomen oder einen Alkenylrest mit 2 bis 5 Kohlenstoffatomen bedeuten, und ihre physilogisch verträglichen Säureadditionssalze.

2. 3-Hydrazino-6-(2-methyl-imidazol-1-yl)-pyridazin.
3. 3-Hydrazino-6-(2-äthyl-imidazol-1-yl)-pyridazin.
4. 3-(2-Isopropyliden-hydrazino)-6-(2-methyl-imidazol-1-yl)-pyridazin.
5. 3-(2-Isopropyliden-hydrazino)-6-(2-äthyl-imidazol-1-yl)-pyridazin.

6. Verfahren zur Herstellung von Verbindungen der Formel I, gemäß Anspruch 1 dadurch gekennzeichnet, daß man das Alkali- oder Erdalkalimetall-Salz eines am N-Atom 1 unsubstituierten Imidazols entsprechend dem Rest R der Formel I in Anspruch 1 in einem hochsiedenden polaren aprotischen Lösungsmittel bei Temperaturen von 20 bis 170°C mit 3,6-Dichlorpyridazin umsetzt und das erhaltene 3-Chlor-6-(imidazol-1-yl)-pyridazin anschließend mit Hydrazinhydrat gegebenenfalls in Gegenwart eines Lösungsmittels bei Temperaturen von 80°C bis zu Rückflußtemperaturen des Reaktionsgemisches zu einem 6-(Imidazol-1-yl)-3-hydrazino-pyridazin der allgemeinen Formel I umsetzt und gegebenenfalls die erhaltene Hydrazinoverbindung mit einer Carbonylverbindung der Formel

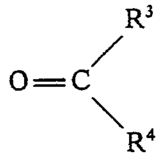

in der $R^3$ und $R^4$ die oben genannten Bedeutungen haben, zu einem Hydrazon umsetzt und gegebenenfalls die erhaltene Verbindung der Formel I in ein Säureadditionssalz einer physiologisch verträglichen Säure überführt.

7. Therapeutisches Mittel, enthaltend neben üblichen Träger- und Verdünnungsmitteln eine Verbindung der Formel I oder deren physiologisch verträgliches Säureadditionssalz als Wirkstoff.

8. Verbindung der Formel I nach Anspruch 1 als Arzneimittel zur Verwendung bei der Behandlung der Hypertonie.

**Claims**

1. 6-Imidazolyl-3-hydrazino-pyridazines of the formula I

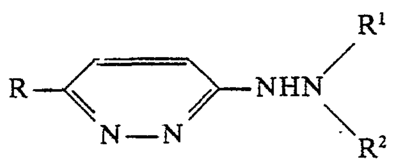

(I)

where R is an imidazole radical which is unsubstituted or possesses one or two halogen substituents or one, two or three $C_1-C_3$-alkyl substituents, and $R^1$ and $R^2$ are each hydrogen or together are a radical

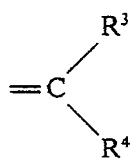

where $R^3$ and $R^4$ are each lower alkyl of 1 to 4 carbon atoms or alkenyl of 2 to 5 carbon atoms, and their physiologically tolerated addition salts with acids.

2. 3-Hydrazino-6-(2-methyl-imidazol-1-yl)-pyridazine.

3. 3-Hydrazino-6-(2-ethyl-imidazol-1-yl)-pyridazine.

4. 3-(2-Isopropylidene-hydrazino)-6-(2-methyl-imidazol-1-yl)-pyridazine.

5. 3-(2-Isopropylidene-hydrazino)-6-(2-ethyl-imidazol-1-yl)-pyridazine.

6. A process for the preparation of a compund of the formula I as claimed in claim 1, wherein the alkali metal salt or alkaline earth metal salt of an imidazole which is unsubstituted at the N-atom 1 and corresponds to the radical R in the formula I of claim 1 is reacted with 3,6-dichloropyridazine in a high-boiling polar aprotic solvent at from 20 to 170°C and the 3-chloro-6-(imidazol-1-yl)-pyridazine obtained is then reacted with hydrazine hydrate, in the presence or absence of a solvent, at from 80°C to the reflux temperature of the reaction mixutre, to give a 6-(imidazol-1-yl)-3-hydrazino-pyridazine of the general formula I, which may or may not then be reacted with a carbonyl compound of the formula

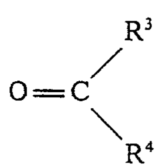

where $R^3$ and $R^4$ have the above meanings, to give a hydrazone, the compound obtained, of the formula

I, being converted, if desired, to an addition salt with a physiologically tolerated acid.

7. A therapeutic agent which contains a compound of the formula I, or a physiologically tolerated addition salt thereof with an acid, as the active compound, in addition to conventional exicpients and diluents.

8. A compound of the formula I as claimed in claim 1 for use as a drug in the treatment of hypertonia.

**Revendications**

1. 6-Imidazolyl-3-hydrazino-pyridazines de la formule

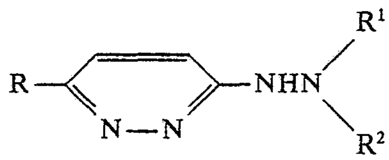

(I)

dans laquelle R désigne un reste imidazolyle pouvant être mono- ou di-substitué par des atomes d'halogène ou mono- à tri-substitué par des radicaux alcoyle en $C_1$ à $C_3$ et $R^1$ et $R^2$ représentent chacun un atome d'hydrogène ou forment ensemble un groupe

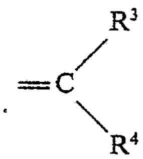

dans lequel $R^3$ et $R^4$ désignent chacun un radical alcoyle inférieur en $C_1$ à $C_4$ ou alcényle en $C_2$ à $C_5$, ainsi que leurs sels d'addition d'acides physiologiquement acceptables.

2. La 3-hydrazino-6-(2-méthyl-imidazol-1-yl)-pyridazine.

3. La 3-hydrazino-6-(2-éthyl-imidazol-1-yl)-pyridazine.

4. La 3-(2-isopropylidène-hydrazino)-6-(2-méthyl-imidazol-1-yl)-pyridazine.

5. La 3-(2-isopropylidène-hydrazino)-6-(2-éthyl-imidazol-1-yl)-pyridazine.

6. Procédé de préparation de composés de la formule I selon la revendication 1, caractérisé en ce que l'on fait réagir dans un solvant aprotique polaire à point d'ébullition élevé, entre 20 et 170° C, de la 3,6-dichloro-pyridazine avec un sel de métal alcalin ou alcalino-terreux d'un imidazole non substitué sur l'atome d'azote 1, correspondant au reste R dans la formule I de la revendication 1, puis on transforme la 3-chloro-6-(imidazol-1-yl)-pyridazine obtenue par réaction avec l'hydrate d'hydrazine, entre 80° C et la température de reflux du mélange réactionnel et éventuellement en présence d'un solvant, en une 6-(imidazol-1-yl)-3-hydrazino-pyridazine de la formule générale I, le dérivé hydrazino formé pouvant, si on le désire, être transformé par réaction avec un composé carbonylé de la formule

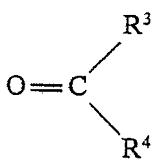

dans laquelle $R^3$ et $R^4$ possèdent les significations définies plus haut, en une hydrazone et le composé de la formule I obtenu en un sel d'addition d'un acide physiologiquement acceptable.

7. Composition thérapeutique, contenant comme principe actif un composé de la formule I ou un sel d'addition d'un acide physiologiquement acceptable d'un tel composé à côté de véhicules et diluants usuels.

8. Utilisation d'un composé de la formule I selon la revendication 1 comme médicament pour le traitement des états d'hypertension.